# EUROPEAN PATENT APPLICATION

(11) **EP 3 682 810 A1**
(43) Date of publication of application: **22.07.2020**
(21) Application number: 19151800.0
(22) Date of filing: 15.01.2019
(51) Int. Cl.: A61B 8/06, A61B 8/12, A61B 8/00, G01S 15/89, G01S 7/52

(54) **INTRAVASCULAR ULTRASOUND DEVICE**

(71) Applicant: Koninklijke Philips N.V., 5656 AG Eindhoven (NL)
(72) Inventor: PEKAR, Martin, 5656 AE Eindhoven (NL); MIHAJLOVIC, Nenad, 5656 AE Eindhoven (NL); VAN RENS, Antonia Cornelia, 5656 AE Eindhoven (NL)
(74) Representative: de Haan, Poul Erik

(57) **Abstract**

An intravascular ultrasound device (12) comprising an array (16) of ultrasound transducer elements (18) which extends in a longitudinal (22) and annular (20) direction along and around the device body (14). The device includes control electronics which allow the array to driven either as a set of longitudinally oriented (28) subgroups of elements (e.g. each including one or more rows of the array), or as a set of annularly oriented subgroups (26) of elements (e.g. each including one or more columns of the array). By switching between these two different operation modes, two different functions can be performed using the same single unitary array of elements: either cross-sectional imaging in the longitudinal mode, or blood flow sensing in the annular mode.

## Description

### FIELD OF THE INVENTION

The present invention relates to an intravascular ultrasound device.

### BACKGROUND OF THE INVENTION

An array of transducer elements may be used when imaging is desired in a confined space.

One key example is for intravascular ultrasound (IVUS) imaging. This is widely used in interventional cardiology as a diagnostic tool for a diseased vessel, such as an artery, within the human body to determine the need for treatment, to guide an intervention, and/or to assess its effectiveness. To perform an IVUS imaging study, an IVUS catheter that incorporates one or more ultrasound transducers is passed into the vessel and guided to the area to be imaged.

The transducers emit and receive ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by a transducer and passed to an IVUS imaging system, which is connected to the IVUS catheter by way of a patient interface module (PIM). The imaging system processes the received ultrasound signals to produce a cross-sectional image of the vessel where the device is placed.

Blood flow is an important clinical parameter, for instance for clinical assessment of stenosis (narrowing of a body vessel). State-of-the-art intravascular ultrasound devices used to assess geometry of such a stenosis are not able to provide information on blood flow (i.e. blood flow speed, volumetric flow rate) because the orientation of the transducer element array is always perpendicular to the cross-section of the vessel (i.e. parallel with a longitudinal axis of the device), and thus parallel to the blood flow. This orientation is needed to achieve cross-sectional imaging of the blood vessel, but it is not suitable for flow measurement.

Devices are known which are capable of performing both cross-sectional imaging and blood flow measurement, but these require provision of separate arrays of transducer elements for performing the two functions, each with a different orientation or alignment. These are controlled with separate electronics to perform the two functions. However, this makes the devices more complex electronically, and also necessitates a larger number of components and a larger overall sensing area (footprint) on the device. This increases form factor. Alternatively, sensing area can be reduced, but at the cost of ultrasound imaging or sensing power.

There is hence required an improved intravascular ultrasound imaging device, capable of performing both cross-sectional imaging, and blood flow measurement.

### SUMMARY OF THE INVENTION

The invention is defined by the claims.

According to examples in accordance with an aspect of the invention, there is provided an intravascular ultrasound device, the device having a body for intravascular insertion, and the device comprising:
a two-dimensional array of transducer elements, comprising columns extending annularly around a circumference of the device body, and comprising rows extending along a direction of a longitudinal axis of the device body, and
driving electronics permitting independent control of each of a plurality of annularly aligned subgroups of the transducer elements, and each of one or more longitudinally aligned subgroups of the transducer elements;
and control means permitting driving of the array
   in an annular mode for generating ultrasound signals along one or more annularly aligned planes; or
   in a longitudinal mode for generating ultrasound signals along one or more longitudinal planes.

The invention is based on using a single, unitary 2D array of ultrasound transducer elements and controlling elements of the array in different geometrical configurations for sensing blood flow and for performing cross-sectional imaging respectively. The elements can be controlled in different configurations for producing annularly directed ultrasound signals in one mode and longitudinally directed ultrasound signals in another. Signals directed parallel to annular planes can be used for sensing blood flow, since these are directed along planes perpendicular to the direction of the blood flow. Signals directed parallel with longitudinally aligned planes can be used for cross-sectional imaging, since these are directed along planes perpendicular to a cross-section of the blood vessel lumen in which the device is inserted.

The invention hence provides a simplified construction, since only a single array is used. In embodiments, this allows for instance for only a single set of connecting wires to be used, which may greatly simplify design, and manufacture. Furthermore, the imaging or sensing power achievable in each mode is enhanced since the whole array of transducer elements can be used in each mode. In one mode, the array may be controlled as a set of longitudinally aligned subgroups of elements for instance e.g. each comprising one or more rows. In the other mode, the array may be controlled as a set of annularly aligned subgroups of elements for instance, e.g. each comprising one or more columns.

The generated signals are emitted from the device along paths located in the one or more annularly aligned planes (for the annular control mode) or the one or more longitudinally aligned planes (for the longitudinal control mode).

Annularly aligned means having an annular or part annular shape; originating from an annular or part annular section or region of the ultrasound device outer surface for instance. Part annular means that the plane does not describe a complete closed annulus (loop) but only a section or set of sections of an annulus. The annularly aligned planes can be tilted to some extend toward the longitudinal axis; they need not be parallel to a radial axis. Longitudinally aligned means originating from a linear or longitudinal section or region of the ultrasound device; it may tilted to some extend toward the radial axis.

The 2D array may extend fully or only partially around a circumference of the device body, i.e. the array need not describe a complete closed annulus shape. This is true also of the annularly aligned planes.

Each subgroup of elements may be electrically connected together in a chain. Each subgroup may be independently drivable or addressable via application of a drive signal along this chain.

The transducer elements may be arranged at an outer surface of the ultrasound device.

The annular mode may comprise controlled operation of the annular subgroups. The longitudinal mode may comprise controlled operation of the longitudinal subgroups. Hence, in this case, the annular mode comprises use of the annular subgroups and the longitudinal mode comprises use of the longitudinal subgroups.

The respective modes may comprise use of at least a portion the annular subgroups and longitudinal subgroups respectively.

The annular mode may comprise use only of the annular subgroups. The longitudinal mode may comprise use only of the longitudinal subgroups.

In examples, the annular mode may be for implementing cross-sectional imaging. The longitudinal mode may be for implementing blood flow sensing.

There are different ways in which each of these functions may be performed, as will be explained further below. As noted above, signals directed parallel to annular planes can be used for sensing blood flow, since these are directed along planes perpendicular to the direction of the blood flow. Signals directed parallel with longitudinally aligned planes can be used for cross-sectional imaging, since these are directed along planes perpendicular to a cross-section of the blood vessel lumen in which the device is inserted.

Each of the plurality of annularly aligned subgroups of transducer elements may comprise one or more of the columns of the array. Each of the plurality of longitudinally aligned subgroups of the transducer elements may comprise one or more of the rows.

Each subgroup may comprise only a section or portion of the respective rows or columns that it includes in some cases, or may comprise the whole of the rows or columns.

The annularly aligned subgroups and the longitudinally aligned subgroups may intersect in a grid-type arrangement, and with transducer elements at the intersections being shared (by the two subgroups that are intersecting).

In some examples, the driving electronics may comprise matrix addressing circuitry permitting independent addressing of subsets of one or more of the elements of the array. The subsets may fall at the intersections of the annular and longitudinal subgroups of elements.

According to some embodiments, each transducer element, or each of a plurality of subsets of the transducer elements, may be addressable by two control signal components, a first and second control signal component, for putting the respective element into a certain driving mode (for signal transmission or reception). Signal transmission or reception may be performable by an element only when in said mode, or signal transmission or reception at a certain higher power level may be performable when in that mode.

The driving electronics may include sets of components for independently supplying to each element first and second control signal components.

The driving mode may be a mode for generating or sensing ultrasound signals at some minimum or threshold power level, e.g. a collapse mode in the case that CMUT transducers are used.

For example, according to one set of embodiments, each transducer element, or each of a plurality of subsets of elements, is adapted to become activated in a certain driving mode only upon application of a bias control voltage by a bias control circuit and simultaneous stimulation by a transmit circuit or receive circuit; and
wherein each of the annular subgroups of elements, or each of the longitudinal subgroups of elements, is electrically connected to a respective transmit and receive circuit, and wherein each of the other of the annular or longitudinal subgroups is connected to a respective bias circuit.

Elements or subgroups of elements at the intersections of the annular and longitudinal subgroups are hence individually addressable (for putting them into said certain driving mode). The bias control circuits and transmit/receive circuits may form components of the driving electronics and provide (part of) a matrix addressing circuit in this case.

Here the driving electronics comprises a plurality of transmit and receive circuits and plurality of bias control circuits. The transmit and receive circuits may be combined into integrated transmit/receive circuits for facilitating both the functions of signal transmission and signal reception. The certain driving mode become activate only upon stimulation by a transmit/receive circuit and simultaneous application of a bias control voltage for instance.

The bias control circuits are each configured to apply a bias voltage to the respective subgroup of elements to which it is connected.

Individual elements or subsets of elements falling at the intersections of the annular subgroups and longitudinal subgroups of elements are each independently addressable by activation of the respective bias control circuit and transmit/receive circuit feeding the respective intersecting subgroups.

The annular control mode may comprise sequentially activating at least a portion of the annular subgroups of transducer elements.

The respective annular subgroups may be sequentially activated in a longitudinal direction (longitudinal activation path).

Activating an annular subgroup may mean driving the annular subgroup of elements to perform ultrasound sensing, e.g. to generate and/or receive ultrasound signals. The mode of operation permits sensing or measurement of blood flow, for instance via a speckle tracking method. Flowing blood can be followed by the sequentially activating subgroups of elements, and a flow speed determined based on knowledge of a distance between respective subgroups and a timing of a detection of a given scatterer or speckle pattern at each subgroup.

The annular control mode may comprise driving at least a portion of the annular subgroups of transducer elements with a controlled pattern of pulses and delays for implementing beam steering in a part-longitudinal direction, for implementing Doppler measurement of blood flow speed along the longitudinal direction.

Part longitudinal direction means for instance within an annular or part-annular plane oriented or tilted partly toward the longitudinal axis, i.e. along an annular plane or section of an annular plane oriented in a direction part way between the radial axis and a longitudinal axis of the device body.

According to one or more examples, the annular control mode may comprise a speckle tracking method, for instance for measuring blood flow, the method comprising tracking of speckle motion between longitudinal locations of two or more of the annular subgroups. A speckle point, or element, or pattern, may be detected at two or more annular locations and based on known longitudinal separation between the respective annular subgroups of elements, a blood flow speed determined.

According to one or more examples, the annular control mode may comprise controlling the plurality of annular subgroups to generate an ultrasound wave output pattern or profile having at least two longitudinally displaced regions of relative high sensitivity, and tracking the movement of an element or point within flowing blood between said two longitudinal locations of maximal sensitivity. This approach may make use for instance of apodization to virtually split the active array into two longitudinally adjacent sub-apertures, each of which is sensitive at a different longitudinal location along the device body.

The longitudinal control mode may comprise sequential driving of at least a portion of the longitudinal subgroups of transducer elements.

The control means may be configured in use to derive a measure of a blood flow speed in a longitudinal direction using the annular control mode, and/or is configured to derive a measure of a cross-sectional area of a lumen in which the device is positioned using the longitudinal control mode.

The control means may be further configured in use to derive a measure of volumetric blood flow using the derived measures of blood flow speed and cross-sectional area.

According to one or more sets of embodiments, each of the transducer elements may comprise one or more CMUT transducer cells.

According to one set of embodiments, the device may comprise a catheter.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a section of an example ultrasound device according to one or more embodiments;
Fig. 2 illustrates a transducer element array as may be included in one or more embodiments;
Fig. 3 shows a further view of the example ultrasound device of Fig. 1;
Figs. 4-6 illustrate various possible annular and longitudinal control modes according to one or more embodiments;
Fig. 7 illustrates generation of annularly and longitudinally aligned planes in example annular and longitudinal control modes respectively;
Fig. 8 shows an example matrix addressing scheme for implementing control of the array of transducer elements;
Fig. 9 illustrates use of the scheme of Fig. 8 for example annular and longitudinal control modes;
Fig. 10 illustrates one example annular control mode for implementation in one or more embodiments, for measuring blood flow speed, based on a scatterer-tracking approach;
Fig. 11 illustrates a further annular control mode option for measuring blood flow speed, based on use of apodization;
Fig. 12 illustrates a further annular control mode option for measuring blood flow speed, based on a Doppler ultrasound approach; and
Fig. 13 illustrates an example longitudinal control mode according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an intravascular ultrasound device comprising an array of ultrasound transducer elements which extends in a longitudinal and annular direction along and around the device body. The device includes control electronics which allow the array to driven either as a set of longitudinally oriented subgroups of elements (e.g. each including one or more rows of the array), or as a set of annularly oriented subgroups of elements (e.g. each including one or more columns of the array). By switching between these two different operation modes, two different functions can be performed using the same single unitary array of elements: cross-sectional imaging in the longitudinal mode, or blood flow sensing in the annular mode.

One advantageous application for embodiments of the present invention is for the measurement of blood flow.

Blood flow is an important clinical parameter, for instance for clinical assessment of stenosis (narrowing of a body vessel). State-of-the-art intravascular ultrasound (IVUS) devices used to assess geometry of such a stenosis are not able to provide information on blood flow (i.e. blood flow speed, volumetric flow rate) because the orientation of the transducer element array is always perpendicular to the cross-section of the vessel (i.e. parallel with a longitudinal axis of the device), and thus parallel to the blood flow. This orientation is needed to achieve cross-sectional imaging of the blood vessel, but it is not suitable for flow measurement.

The present invention is aimed at providing an IVUS device which can perform both cross-sectional imaging and blood flow measurement in a compact form factor.

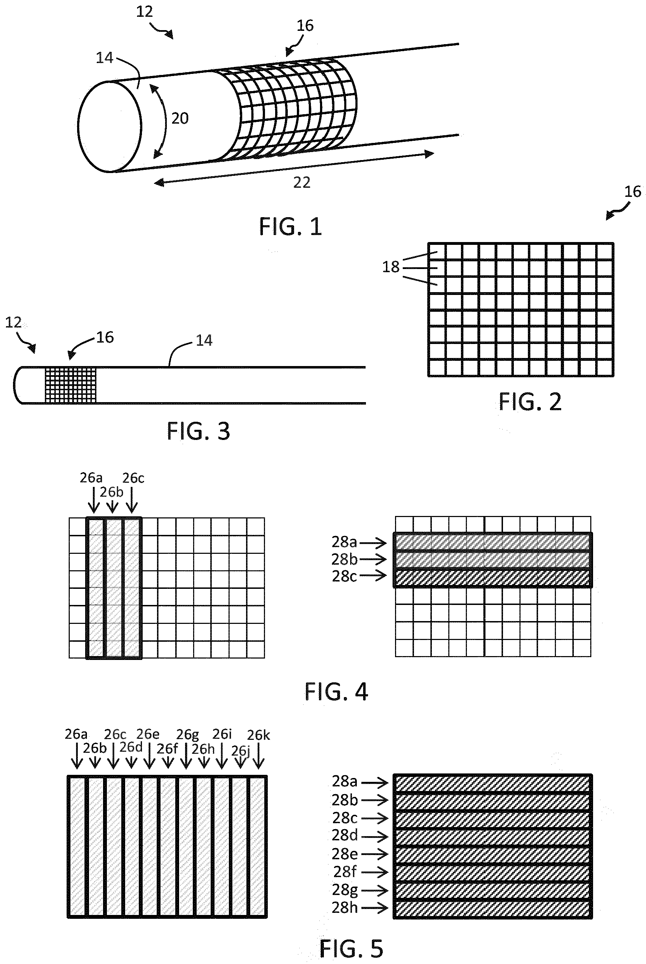

Fig. 1 illustrates an example intravascular ultrasound (IVUS) device 12 in accordance with one or more embodiments of the invention. Fig. 2 shows the example 2D array of ultrasound elements comprised by the device. Fig. 3 shows a side elevation view of the IVUS device 12 with the array in position.

The device 12 has an elongate body 14 for intravascular insertion, e.g. insertion within an artery, vein or any other blood vessel.

The device 12 comprises a two-dimensional array 16 of transducer elements 18. The array comprises columns extending annularly around a circumference of the device body, and rows extending longitudinally along the device, parallel to a longitudinal axis of the device body. The longitudinal 22 and annular 20 axes respectively of the device body are indicated in Fig. 1.

In some examples, each of the transducer elements 18 may comprise one or more CMUT (capacitive micromachined ultrasound transducer) cells. Any other kind of transducer may in general alternatively be used.

For specific embodiments (discussed below) in which transducers are adapted to receive and be sensitive to first and second control signal components, for instance a bias signal and drive signal, transducers having this dual signal responsive functionality may be required. For other embodiments, any suitable ultrasound transducers may be used.

The transducer elements may be contiguously positioned as in Figs. 1-3, or there may be some separation or spacing between neighboring transducer elements 18 in other examples (e.g. for heat dissipation).

The 2D array 16 may extend annularly around the whole circumference of the device body 14, or only around one portion or section of it. It may optionally comprise a plurality of discontinuous annular sections, which may be annularly spaced from one another in some cases (with an annular spacing separating them).

The IVUS device 12 further includes driving electronics (not shown in Figs. 1-3) permitting independent control of each of a plurality of annularly aligned subgroups 26 of the transducer elements 18, and each of a plurality of longitudinally aligned 28 subgroups of the transducer elements.

Control means are provided permitting driving of the array 16
in an annular mode for generating ultrasound signals emitted in one or more annularly aligned planes; or
in a longitudinal mode for generating ultrasound signals emitted in one or more longitudinal planes.

The control means may be a controller, for instance comprising a processor, or may comprise circuitry for controlling driving of the array in the different modes. The control circuitry and driving circuitry may be integrated as a single set of circuitry in some examples.

The control means may comprise an application specific integrated circuit (ASIC).

The control circuit allows for electronic switching between the annular (short axis) and the longitudinal (long axis) control modes.

The arrangement hence effectively provides a bi-plane annular array of ultrasound elements which can be controlled in different geometrical arrangements to achieve blood flow sensing in one configuration (annular mode) or cross-sectional imaging in the other configuration (the longitudinal mode).

The annular mode preferably comprises controlled operation of the annular subgroups of the transducer elements 18, and the longitudinal mode preferably comprises controlled operation of the longitudinal subgroups of transducer elements 18. Figs. 4-6 illustrate example arrangements of subgroups of transducer elements in accordance with various possible embodiments.

Fig. 4 illustrates example subgroups according to one embodiment. Fig. 4 (left) illustrates an example set of three annular subgroups 26a, 26b, 26c, and Fig. 4 (right) illustrates an example set of three longitudinal subgroups 28a, 28b, 28c. In this example, each annular subgroup comprises a single column (annular line) of transducer elements, and each longitudinal subgroup comprises a single row (longitudinal line) of transducer element. The longitudinal 28 and annular 26 subgroups of elements intersect at right angles and share elements where they intersect.

The control means allows operating the device either as the set of annular subgroups 26 (so that each annular subgroup is individually addressable) or as the set of longitudinal subgroups 28, so that each longitudinal subgroup is individually addressable. Switching is performable between control of the annular and longitudinal groups.

Although in Fig. 4 only three annular and longitudinal subgroups are shown respectively, this is for purposes of illustration only.

Fig. 5 shows a more preferred arrangement of annular 26 and longitudinal 28 subgroups of transducer elements 18 for use in annular and longitudinal control modes respectively. Here, the annular subgroups 26a-26k span the whole longitudinal length of the 2D array 16, and the longitudinal subgroups 28a-28h span the whole annular height of the 2D array 16. The array may be controlled as the set of annular subgroups 26 of elements in the annular mode, and as the set of longitudinal subgroups 28 in the longitudinal mode.

Fig. 5 (left) and Fig. 5 (right) represent the annular and longitudinal control modes respectively, with the annular control mode permitting controlled operation of the array as a set of annularly aligned subgroups of elements, and the longitudinal control mode permitting control of the array as a set of longitudinally aligned subgroups of transducer elements.

In this example, each annular subgroup comprises a single column (annular line) of elements, and each longitudinal subgroup comprises a single row (longitudinal line) of elements.

However, this is not essential, and in other examples, each of the annular subgroups and/or each of the longitudinal subgroups of elements may comprise more than one column or more than one row respectively. The annular subgroups can comprise a different number of columns as the number of rows comprised by each longitudinal subgroup. Furthermore, different of the annular subgroups can have different numbers of columns, and different of the longitudinal subgroups can have different numbers of rows.

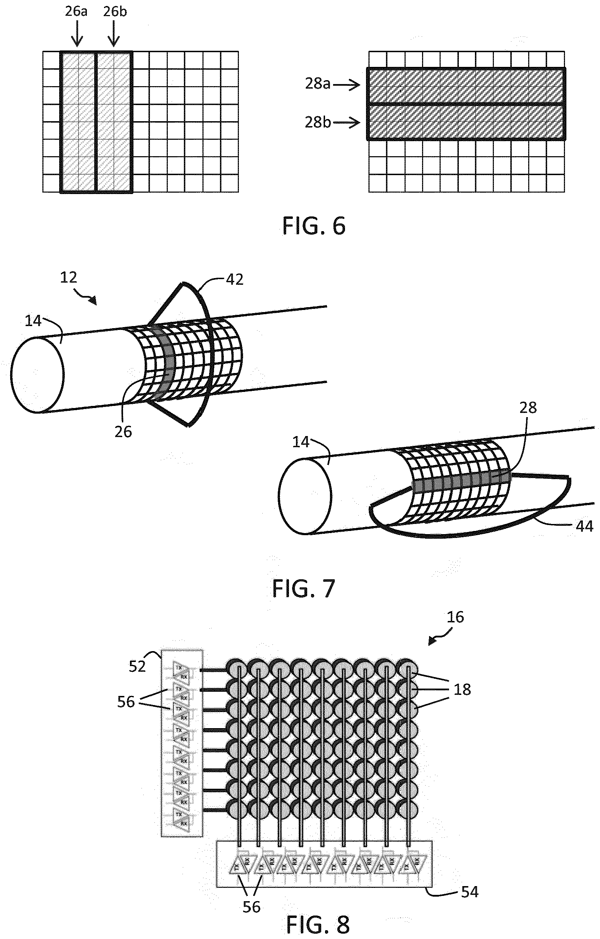

By way of illustration, Fig. 6 shows one example in which the annular subgroups 26 of elements each comprise two columns and the longitudinal subgroups of elements 28 each comprise two rows. Only two annular and longitudinal subgroups are shown, but this is for illustration only; any number of each can be included.

Furthermore, each annular subgroup 26 and longitudinal subgroup 28 may comprise only a portion of one or more columns or rows respectively, e.g. just a section of each of one or more rows or columns, or every other element in one or more rows or columns for instance.

The control means permit driving of the array 16 in an annular mode for generating ultrasound signals along one or more annularly aligned planes, or in a longitudinal mode for generating ultrasound signals along one or more longitudinally aligned planes.

An example is schematically illustrated in Fig. 7. Fig. 7 (top) represents an example annular control mode, and Fig. 7 (bottom) represents an example longitudinal control mode. As shown, in the annular control mode, ultrasound signals can be generated along one or more annularly aligned planes 42. In the longitudinal control mode, ultrasound signals can be generated in one or more longitudinally aligned planes 44.

For example, ultrasound signals can be generated in annularly aligned planes by controlled driving of annularly aligned subgroups of elements. This is shown in Fig. 7 where an annular subgroup 26 of elements is being driven to generate signals in an annularly aligned plane 42. Ultrasound signals can be generated in longitudinally aligned planes 44 by controlled driving of longitudinally aligned subgroups of elements. This is also shown in Fig. 7 (bottom) where a longitudinal subgroup 28 of elements is being driven to generate signals in a longitudinally aligned plane 44.

The annular plane 42 originates from an annular region of the IVUS device.

The longitudinal plane 44 originates from a longitudinally aligned region of the IVUS device.

The generated ultrasound signals need not fill or completely occupy a whole plane, they may simply be generated oriented or aligned parallel with such a plane. They are emitted along paths lying in such planes.

The annular control mode may comprise driving only subsets of one or more of the annular subgroups at a time to generate and/or sense ultrasound signals. For instance, annular subgroups may be activated in this way only one at a time, for instance in sequence, along a (longitudinally oriented) sequential activation path.

Likewise, the longitudinal control mode may comprise driving only subsets of one or more of the longitudinal subgroups at a time to generate and/or sense ultrasound signals. For instance, longitudinal subgroups may be activated in this way only one at a time, for instance in sequence, along an (annularly oriented) sequential activation path.

Alternatively, in either control mode, subgroups can be driven concurrently or simultaneously in some examples, for instance for purposes of beamforming or shaping or beam-steering, or to generate a particular wave profile.

The driving electronics can be implemented in a variety of different ways and different examples will be described below.

A first example configuration comprises providing a separate driver and receiver circuit pair connected to each respective annular subgroup of elements and to each respective longitudinal subgroup of elements to permit individual addressing of each. This provides a form of matrix addressing scheme.

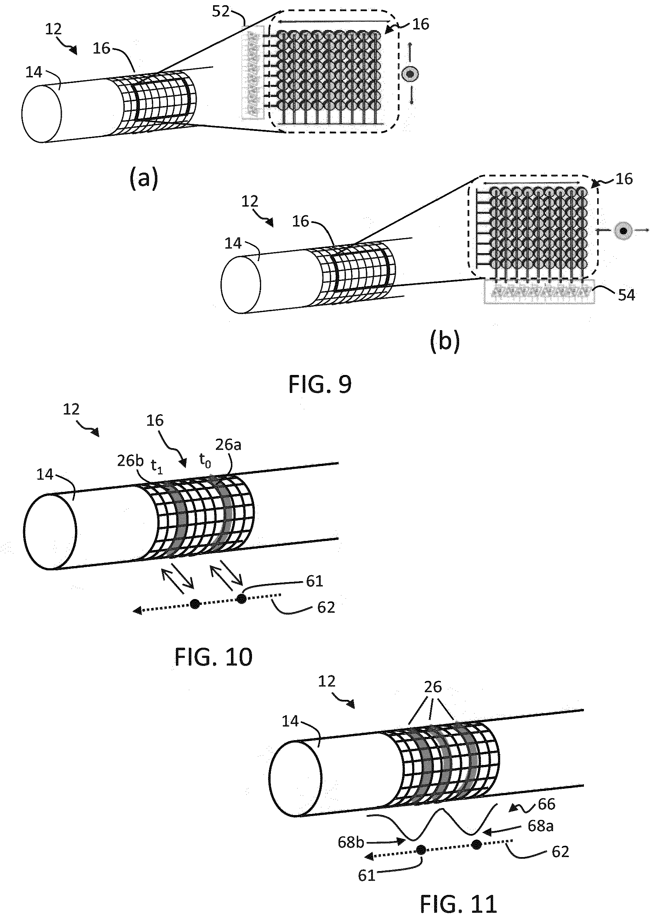

An example circuitry is shown in Fig. 8 and Fig. 9 for example. The 2D array 16 of ultrasound elements comprises rows and columns, where all of the elements along each row are electrically connected together in a respective chain, and all of the elements in each column are electrically connected together in a chain. A first 52 and second 54 set of driver circuit (TX) and receiver circuit (RX) pairs 56 are provided for connecting to the rows and columns respectively. Each row is connected to a unique TX/RX circuit pair, and each column is connected to a unique TX/RX circuit pair.

The array 16 of transducer elements is shown. The array in general comprises N*M transducer elements, N being the number of elements included per row, M being the number of elements included per column.

Each of the transducer elements 18 (which may in come examples be individual CMUT cells) has two electrodes, a top electrode (shown in light grey) which may for instance be integrated with a flexible membrane covering of the IVUS device and a bottom electrode (shown in dark grey) which may for instance be coupled atop a rigid substrate comprised by an outer rigid surface of the IVUS device body 14.

The top electrodes of the transducer elements 18 are shorted per column, the bottom electrodes of the transducer elements 18 are shorted per row. This effectively provides N annular subgroups of elements and M longitudinal subgroups of elements.

By actively driving the first set 52 of TX/RX circuits 56 while grounding the column circuitry, longitudinal mode is achieved as illustrated schematically in Fig. 9(a). By actively driving the second set 54 of TX/RX circuits 56 while grounding the row circuitry, the annular mode is achieved as illustrated schematically in Fig. 9(b).

In accordance with a further example set of embodiments, a different driving scheme may be used.

According to this set of examples, each transducer element 18, or each of a plurality of subsets of the transducer elements, may addressable by two control signal components, a first and second control signal component, for putting the respective element into a certain driving mode (for signal transmission or reception). Signal transmission or reception may be performable by an element only when in said mode, or signal transmission or reception at a certain higher power level may be performable when in that mode.

The driving electronics may include sets of components for independently supplying to each element first and second control signal components.

The driving mode may be a mode for generating or sensing ultrasound signals at some minimum or threshold power level, e.g. a collapse mode in the case that CMUT transducers are used.

For example, according to one set of embodiments, each transducer element, or each of a plurality of subsets of elements, is adapted to become activated in a certain driving mode only upon application of a bias control voltage by a bias control circuit and simultaneous stimulation by a transmit or receive circuit; and
wherein each of the annular subgroups of elements, or each of the longitudinal subgroups of elements, is electrically connected to a respective transmit and receive circuit, and wherein each of the other of the annular or longitudinal subgroups is connected to a respective bias circuit.

Elements or subgroups of elements at the intersections of the annular and longitudinal subgroups are hence individually addressable (for putting them into said certain driving mode). The bias control circuits and transmit/receive circuits provide (part of) a matrix addressing circuit in this case.

Here the driving electronics comprises a plurality of transmit and receive circuits and plurality of bias control circuits.

The bias control circuits are bias control circuits are each configured to apply a bias voltage to the respective subgroup of elements to which it is connected.

Individual elements or subgroups of elements falling at the intersections of the annular subgroups and longitudinal subgroups of elements are each independently addressable by activation of the respective bias control circuit and transmit/receive circuit feeding the respective intersecting subgroups.

Where the transducer elements each comprise CMUT cells, the bias voltage supplied by a respective bias control circuit puts the CMUT cell into collapse mode. In collapse mode, the achievable ultrasound transmission and sensing power are significantly enhanced. Hence, the bias voltage transforms each transducer element into a higher power mode. For CMUT cells, typically the higher power (collapse) mode is significantly higher power than the lower power (non-biased) mode, and hence the bias voltage, in transforming to the collapse mode, may effectively act as an ON/OFF switch for the transducer element.

In other examples for instance, the two different control signal components may each be for contributing a respective power component. For instance both may provide the same active driving signal, in exact parallel or synchrony with one another, and by applying only one, a first lower power level mode is provided, and by applying both, a second higher power level mode is achieved.

In one simple example, the array may include control electronics permitting independent driving of each of only two different annular subgroups. A first annular subgroup covers one longitudinal half of the array and a second annular subgroup covers the other longitudinal half of the array. The elements of each annular subgroup are connected together electrically in respective chains. Each of the annular subgroups is connected to a respective bias control circuit, allowing for independent biasing of each annular subgroup.

Any desired number of longitudinal subgroups may be provided by providing the corresponding number of transmit and receive circuits, each connected to each respective longitudinal subgroup. By way of example, each row may be connected to a respective transmit and receive circuit allowing for independent driving of each row. Each longitudinal subgroup hence comprises a single row of elements in this example.

In the annular control mode, the array can be controlled to generate ultrasound signals along annular planes by providing a bias voltage to only one of the annular subgroups at any one time. The two subgroups may be biased in sequence. For example, the first annular subgroup may be biased, and then the second annular subgroup biased subsequently. Driving each annular subgroup of elements to generate and/or sense ultrasound signals is done using the transmit and receive circuits coupled along each row. All of the transmit and receive circuits may be driven simultaneously when in the annular mode so that the whole annular subgroup is driven to transmit/receive at the same time. Alternatively, only a subset of the transmit/receive circuit may be driven so that only a corresponding subset of the elements of each annular subgroup is driven at one time.

When in the longitudinal control mode, both of the annular control modes are biased, and then individual longitudinal subgroups (e.g. individual rows) can be independently driven to generate or sense ultrasound signals by driving the corresponding transmit and receive circuits connected to those longitudinal subgroups.

In the further examples, a larger number of annular subgroups may be provided for. By way of example, every column may be connected to an independent bias control circuit for supplying a separate bias voltage. In this way, each annular subgroup comprises a single column of the array. This way, each column and each row are independently drivable.

Above have been described various driving electronics schemes permitting the independent driving of a set of annularly aligned subgroups and a set of longitudinally aligned subgroups of transducer elements respectively.

There will now be described example control schemes for driving this array arrangement in annular and longitudinal modes, for performing blood flow sensing, and cross-sectional imaging respectively. The control means may be adapted to perform any one or more of these different possible control modes.

Annular control modes for blood flow sensing will first be described.

A first example annular control mode comprises controlling the annular subgroups to perform a tracking procedure in which the progress of blood longitudinally past the body of the device is tracked using the plural annular subgroups of transducer elements. For example a particular point or volume element or sub-volume of blood is detected using one or more elements of a first annular subgroup (having a first longitudinal position along the device body) and later detected by at least one further annular subgroup (having a second longitudinal position along the device body). The point detected may be a point scatterer within the blood such as a bubble for instance. By knowing at least the longitudinal displacement between the first annular subgroup and the at least one further annular subgroup, and the respective times at which the point or volume element of blood is detected at the respective annular subgroups, then the speed or velocity of the blood flow (in the lumen in which the device is positioned in use) can be determined.

This technique is illustrated schematically in Fig. 10 which shows control of a first annular subgroup 26a of transducer elements to perform signal generation and sensing at time t₀ to sense or detect an example point scatterer 61 within the blood flow 62. A second annular subgroup 26b of transducer elements is also controlled to perform signal generation and sensing to sense or detect the same example point scatterer 61 within the blood flow 62. The scatterer is detected at the second subgroup 26b at time t₁. These times are recorded and using a known longitudinal distance between the first 26a and second 26b subgroups of elements, the blood flow 62 speed can be determined.

The volume point or element which is tracked may be for instance a bubble or a blood cell, or a particular pattern of blood cells within the blood. In some examples, the first annular subgroup of transducer elements may record an acquired ultrasound reflection or echo pattern attained when transmitting ultrasound signals in their respective annular plane as a characteristic or 'fingerprint' reflection pattern. The control means may then monitor obtained reflection patterns at the at least one further subgroup of transducer elements to test for detection of the same reflection pattern at a later time. When this reflection pattern is detected at the at least one further subgroup of transducer elements, the time at which it is detected at the (or each) further subgroup is recorded, and thus the speed of the blood flow past the device body can be determined.

This technique is often referred to in the art as speckle tracking and is a very well-known method. Speckle tracking usually refers to tracking of multiple point scatterers (e.g. blood cells).

The at least one further subgroup may be controlled to recurrently acquire ultrasound image data during the blood tracking procedure.

In a simplest implementation of this approach, the array may comprise only two annular subgroups of elements, for instance subgroups covering longitudinally half of the 2D array. A larger number of annular subgroups however would achieve more reliable speed measurement results.

A further annular control mode comprises controlling the plurality of annular subgroups to generate an ultrasound wave output pattern or profile having at least two longitudinally displaced regions of relative high sensitivity. This approach makes use of apodization to virtually split the active array into two longitudinally adjacent sub-apertures, each of which is sensitive at a different longitudinal location along the device body. The velocity of blood flow can be determined by tracking the movement of an element or point within flowing blood between these two longitudinal locations of maximal sensitivity.

For instance, by using a number of pulse emissions, the inter-pulse movement can be estimated and the velocity found from the estimated movement and the time between pulses. The procedure is based on the principle of using transverse spatial modulation (longitudinal spatial modulation, i.e. transverse to the annularly aligned planes along which each annular subgroup of elements is configured to generate ultrasound signals) for making the received signal influenced by transverse (longitudinal) motion. Such a transverse modulation can be generated by using apodization on individual transducer array elements together with a special focusing scheme.

The technique is described in detail in J. A. Jensen and P. Munk, "A new method for estimation of velocity vectors," in IEEE Transactions on Ultrasonics, Ferroelectrics, and Frequency Control, vol. 45, no. 3, pp. 837-851, May 1998, and the reader is referred to this document for further technical implementation details.

Fig. 11 schematically illustrates this example procedure. As shown a plurality of the subgroups 26 of elements are controlled to generate an output ultrasound wave profile or distribution 66 which has two locations of peak sensitivity 68a, 68b. The movement of an example scatterer 61 within a blood flow 62 passing the device 12 between these two locations of peak sensitivity can be detected. A distance between the two peak sensitivity locations can be determined based for instance on a theoretical calculation of the estimated wave distribution profile 66 to be generated. By recording a time at which the scatterer is detected at the two locations 68a, 68b, and using the mentioned known separation, a blood flow 62 speed can be sensed.

A further annular control mode comprises a Doppler ultrasound procedure for detecting speed of blood flow. This approach comprises using beam steering to steer ultrasound signals generated by at least a subset of the plurality of annular subgroups of transducer elements partially into the longitudinal direction, so that Doppler tracking blood flow longitudinally alongside the device body can be performed. Beam steering is a very well-known technique in the field of ultrasound sensing, and will not be described in detail here.

In brief, the steering comprises applying delays to ultrasound transmit pulses generated by individual of the annular subgroups of transducer elements, to thereby steer a generated resultant ultrasound beam at a desired angle with respect to the radial direction. This means that there is a component of the generated ultrasound signal which is in the direction of the blood flow, meaning that Doppler tracking can be performed. The steered beam is generated and a frequency shift in the reflected signal is detected. By knowing the angle of the beam relative to the longitudinal direction (and assuming that the blood flow is parallel to the longitudinal direction), and the speed of ultrasound waves in the blood, then from the detected frequency shift, the velocity or speed of the blood flow can be detected using the standard well-known Doppler shift equation.

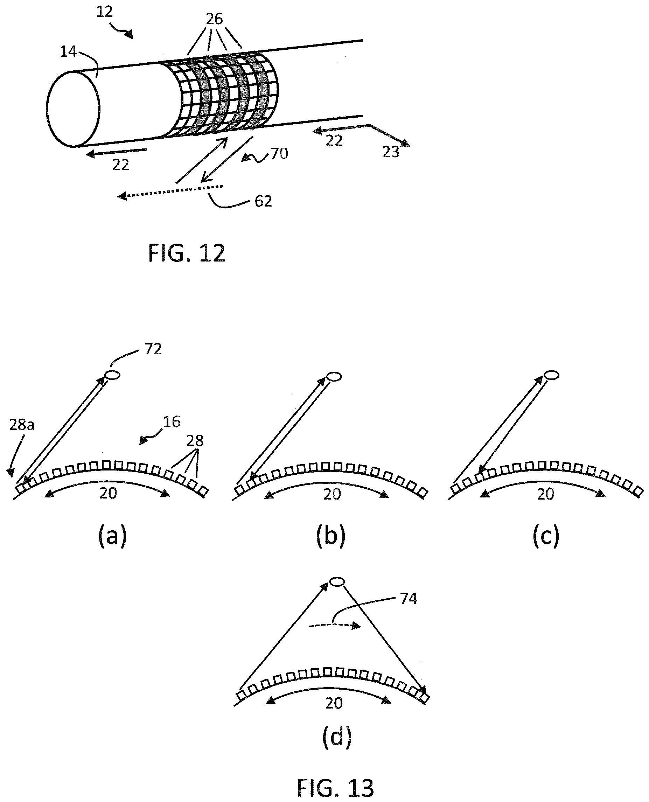

This technique is schematically illustrated in Fig. 12. At least a plurality of the annular subgroups 26 of transducer elements are controlled with delay timings configured to generate a beam 70 which is steered in a direction partially toward the longitudinal axis 22 and partly away from the radial axis 23. The generated steered beam is reflected from passing blood flow 62 and a determined change in the frequency of the sensed reflected signal (compared to the transmitted beam signal) can be used to determine a speed of the blood flow.

Doppler shift techniques are well known in the field of ultrasound sensing and will not be described in detail here. The skilled person will be aware of methods for implementing the above described approach.

It is noted that where in the above description reference is made to controlling a subgroup of transducer elements or to a subgroup of transducer elements generating a signal, this may be taken in general to refer to any subset of one or more of the elements comprised by that subgroup. Preferably, it should refer to a subset of at least two of the transducer elements comprised by the respective subgroup.

Example longitudinal control modes for cross-sectional imaging will now be described.

In general, the longitudinal control mode may comprise control of at least a subset of the longitudinal subgroups 28 of transducer elements to perform signal transmission and receipt along one or more respective longitudinally aligned planes (meaning planes aligned with or originating from a longitudinal subsection or sub-portion of the array 16 or of the device body surface). Each longitudinal subgroup may be controlled to perform both signal generation and transmission, for instance by implementing alternate switching between transmission and sensing modes. Additionally or alternatively, a subset of one or more of the longitudinal subgroups may be controlled to perform only transmission, and with the remaining subgroups (sequentially or otherwise) controlled to perform sensing of the signal reflection.

One example longitudinal sensing procedure, presented by way of illustration is schematically illustrated by Fig. 13(a) - (d).

Fig. 13 shows a schematic cross-sectional view through an example ultrasound device body along a plane perpendicular to the longitudinal axis. The annular direction or axis of the device body is indicated by arrow 20.

The various longitudinal subgroups 28 of elements are shown in cross-section.

According to this example longitudinal control mode, a first longitudinal subgroup 28a of transducer element is controlled to perform signal generation and transmission within a longitudinally aligned plane (a plane aligned with or originating from a longitudinal subsection or line of the array 16 or the device body outer surface). These generated signals fall incident at some target 72. The target may typically be an interior wall of the (e.g. vessel) lumen in which the device is placed in use. Alternatively, it may be any object located circumferentially around the exterior of the device, e.g. a vessel occlusion or a valve or a scatterer within surrounding blood.

According to this example longitudinal control mode approach, the same first 28a longitudinal subgroup of transducer elements is controlled to generate and transmit signals, and the remaining plurality of subgroups 28 of transducer elements are controlled in sequence to perform sensing of the reflected signals. This sequential control is shown by the progression of figures through Figs. 13(a)-(d). The control mode comprises sequentially stepping or walking through the longitudinal subgroups 28 along an annularly directed activation path, as indicated by arrow 74.

This represents just one example. In other examples for instance, the longitudinal subgroups of elements may each perform both transmission of an ultrasound signal and sensing of the reflected signal. The plurality of longitudinal subgroups may be activated sequentially, along an annular activation path, with each sequentially performing signal transmission and subsequently sensing. Ultrasound data may be collected representative of the whole, or a part of, the annular surrounding region around the device, for instance the wall of the lumen in which the device is positioned in use.

This may permit either images to be generated of an annular region surrounding the device, or may permit a radial distance between the device exterior surface and the lumen interior wall to be determined. This distance may be determined at all points circumferentially around the device, by controlling the longitudinal subgroups of elements sequentially all the way annularly around the device. From this a cross-sectional area of the lumen can be determined.

The radial distance can be determined based on a known acoustic speed of the ultrasound waves through the blood, and based on determining a delay between signal transmission and subsequent detection again at the same longitudinal transducer subgroup 28. Based on this speed, and the known delay, the radial distance can be determined.

Ultrasound data collected using any example longitudinal control mode may be processed to generate one or more images. The control means may be configured to perform the image generation, or an additional imaging processing unit may be provided to perform this function. Alternatively, the provided device may perform only data collection, with the device adapted to generate a data output comprising the acquired ultrasound data. This data can be then be used by an external device e.g. an external processor, to generate cross-sectional images, and/or to measure the blood flow speed.

According to an advantageous set of examples, a cross-sectional area of the lumen within which the device is placed may be determined. In particular, the ultrasound data can be used to determine a radial distance between the acoustic output surface of the array of transducer elements and the annular interior wall of the vessel lumen within which the device is placed. This can be determined at all points circumferentially around the device. This can then be used in combination with a known radius of the device itself to determine a total cross-sectional area of the lumen.

Preferably, just the cross-sectional area of the region surrounding the device is calculated, i.e. the whole cross-sectional area minus the device body cross-sectional area. This provides the cross-sectional area of the flow region through which blood flows. If it is known that the device is perfectly (or approximately) centered within the lumen, then this is a simple matter of determining the radial distance, rₐ, between the lumen wall and the device body at any point. If the known device body radius is r_{b} (and the device has a circular cross-sectional area), the cross-sectional area surrounding the device body is given by π(rₐ + r_{b})² - πr_{b}².

If it is not known that the device is perfectly centered, the procedure is slightly more complex. Here, the radial distance between the lumen wall and the device body may be determined at every point around the device circumference (at every longitudinally aligned subgroup 26 of transducer elements). The cross-sectional area of the region surrounding the device body may then be calculated for instance by means of integration (e.g. integrating radial distance with respect to circumferential displacement around the whole circumference of the device body).

Alternatively for instance a radial distance between the device body and the lumen wall at two diametrically opposite points around the device body circumference may be determined, and these added to the known device body diameter, to arrive at an estimated total diameter of the lumen. From this, an approximate cross-sectional area of the whole lumen can be calculated (assuming approximate circularity for instance), and from this subtracted the known cross-sectional area of the device body to yield the cross-sectional area of just the region surrounding the device body (the flow area).

According to advantageous embodiments, the control means may be configured to determine a volumetric flow (volume per second) by determining a blood flow speed using the annular control mode, and determining the cross-sectional area surrounding the device body using the longitudinal control mode, and then multiplying the two together. Thus combining the geometrical information from the cross-sectional imaging mode with the determined flow velocity enables direct calculation of volumetric flow within the blood-vessel.

In contrast to existing bi-planar 2D solutions, embodiments of the present invention enable in at least some examples all array elements to be active in both the longitudinal and in the annular control modes. This is because in each mode, preferably the whole array of elements is controlled either as a set of longitudinally aligned subgroups or as a set of annularly aligned subgroups. The same set of elements is used in both modes; they are simply controlled in different geometrical configurations. All array elements can be activated in transmit and/or in receive mode in both longitudinal and annular modes. As a result, the acoustic performance of the proposed solution is superior to existing solutions.

Embodiments of the invention make use of a control means. According to one or more examples, this may be implemented by a dedicated controller.

A controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An intravascular ultrasound device (12), the device having a body (14) for intravascular insertion, and the device comprising:
a two-dimensional array (16) of transducer elements (18), comprising columns extending annularly around a circumference of the device body, and comprising rows extending along a direction of a longitudinal axis of the device body, and
driving electronics permitting independent control of each of a plurality of annularly aligned subgroups (26) of the transducer elements, and each of one or more longitudinally aligned subgroups (28) of the transducer elements;
and control means permitting driving of the array
in an annular mode for generating ultrasound signals along one or more annularly aligned planes (42); or
in a longitudinal mode for generating ultrasound signals along one or more longitudinal planes (44).

2. The device (12) as claimed in claim 1, wherein the annular mode comprises controlled operation of the annular subgroups (26) and/or
wherein the longitudinal mode comprises controlled operation of the longitudinal subgroups (28).

3. The device (12) as claimed in claim 1 or 2,
wherein the annular mode is for implementing cross-sectional imaging, and/or
wherein the longitudinal mode is for implementing blood flow sensing.

4. The device (12) as claimed in any of claims 1-3, wherein
each of the annularly aligned subgroups (26) of transducer elements comprises one or more of the columns of the array, and/or
wherein each of the longitudinally aligned subgroups (28) of transducer elements comprises one or more of the rows.

5. The device (12) as claimed in any of claims 1-4, wherein the annularly aligned subgroups (26) and the longitudinally aligned subgroups (28) intersect in a grid-type arrangement, and with transducer elements at the intersections being shared.

6. The device (12) as claimed in any of claims 1-5, wherein the driving electronics comprise a matrix addressing circuitry permitting independent addressing of subsets of one or more of the transducer elements (18) of the array, wherein said one or more subsets fall at intersections of the annular (26) and longitudinal (28) subgroups of elements.

7. The device (12) as claimed in any of claims 1-6, wherein each transducer element (18), or each of a plurality of subsets of transducer elements, is adapted to become activated in a certain driving mode only upon application of a bias control voltage by a bias control circuit and simultaneous stimulation by a transmit circuit or receive circuit; and
wherein each of the annular subgroups of elements, or each of the longitudinal subgroups of elements, is electrically connected to a respective transmit and receive circuit, and wherein each of the other of the annular or longitudinal subgroups is connected to a respective bias circuit.

8. The device (12) as claimed in any of claims 1-7, wherein the annular control mode comprises sequentially activating at least a portion of the annular subgroups (26) of transducer elements.

9. The device (12) as claimed in any of claims 1-8, wherein the annular control mode comprises driving at least a portion of the annular subgroups (26) of transducer elements with a controlled pattern of pulses and delays for implementing beam steering in a part-longitudinal direction, for implementing Doppler measurement of blood flow speed in the longitudinal direction.

10. The device (12) as claimed in any of claims 1-9, wherein the annular control mode comprises at least one of:
a speckle tracking method, comprising tracking speckle between longitudinal locations of two or more of the annular subgroups (26); and
controlling the plurality of annular subgroups (26) to generate an ultrasound wave output pattern or profile having at least two longitudinally displaced regions of relative high sensitivity, and tracking the movement of an element or point within flowing blood between said two longitudinally displaced regions of relative high sensitivity.

11. The device (12) as claimed in any of claims 1-10, wherein the longitudinal control mode comprises sequential driving of at least a portion of the longitudinal subgroups of transducer elements.

12. The device (12) as claimed in any of claims 3-11, wherein the control means is configured in use to derive a measure of a blood flow speed in a longitudinal direction using the annular control mode, and/or is configured to derive a measure of a cross-sectional area of a lumen in which the device is positioned using the longitudinal control mode.

13. The device (12) as claimed in claim 12, wherein the control means is further configured in use to derive a measure of volumetric blood flow using the derived measures of blood flow speed and cross-sectional area.

14. The device (12) as claimed in any of claims 1-13, wherein each of the transducer elements comprises one or more CMUT transducer cells.

15. The device (12) as claimed in any of claims 1-14, wherein the device (12) comprises a catheter.
